# EUROPEAN PATENT SPECIFICATION

(11) **EP 4 461 336 B1**
(45) Date of publication and mention of the grant of the patent: **17.06.2026**
(21) Application number: 24202870.2
(22) Date of filing: 20.08.2021
(51) Int. Cl.: A61M 16/06

(54) **HIGH FLOW THERAPY APPARATUS**
THERAPIEVORRICHTUNG MIT HOHEM DURCHFLUSS
APPAREIL DE THÉRAPIE À HAUT DÉBIT

(30) Priority: 01.09.2020 EP 20193976
(43) Date of publication of application: 13.11.2024
(62) Divisional of application: 21769661.6
(73) Proprietor: Stamford Devices Limited, Galway, H91 HE94 (IE)
(72) Inventor: POWER, John, Galway (IE); DUFFY, Aidan, Galway (IE); CASEY, Micheal, Galway (IE)
(74) Representative: Weldon O'Brien Ltd.

(56) References cited:
- WO-A1-2007/058574
- WO-A1-2017/004404
- WO-A2-2005/118036
- WO-A2-2008/116165
- US-A- 5 871 011
- US-A1- 2006 191 537
- US-A1- 2010 122 705
- US-A1- 2014 251 333
- US-B1- 6 736 140
- US-B1- 9 186 474

## Description

### Introduction

The present invention relates to aerosol therapy, especially high flow therapy.

High flow nasal therapy (HFNT) will typically deliver an air/oxygen/aerosol mix to a patient at a rate that exceeds their peak inspiratory rate. An example is 50LPM treatment vs. an average inhalation of about 20 LPM (averaged across the inhalation period of a breath) with a peak inhalation of about 35LPM. Aerosol delivered into the high flow stream will be homogeneously distributed. Therefore, the excess airflow contains aerosolised drug that the patient cannot absorb and this results in reduced efficiency. Also, this excess will disperse into the surrounding room air. This is a fugitive emission that potentially exposes clinicians, patients and visitors to aerosolised drugs and patient-generated pathogens.

Also, during exhalation (typically through the mouth), the high flow therapy may continue to deliver to the nasal cavity. A portion of this flow will travel into the cavity and exit the patient's mouth, and this flow augments the exhalation flowrate and has the potential to collect patient pathogens. The remainder of the flow will exit the cavity via the nostrils, but prior to this it also can collect pathogens.

WO2015/155342A (Stamford Devices Ltd) and WO2019/007950A (Stamford Devices Ltd) describe HFNT systems, in which aerosol is delivered primarily during reduced gas flow periods, in order to increase efficiency and reduce losses. US2012/285455 (Varga et al) describes a mask for patient ventilation. US2004/244799 (Landis) describes a tube seal adapter for face masks. WO2018/204969 (P & M Hebbard PTY) describes a sealing pad for a respiratory mask. WO2019/159063 (Fisher & Paykel) describes a mask which is fitted over a nasal prong interface. Other documents in this field include WO2017/004404), relating to construction of nasal cannulas. US9,186,474 discloses a multi-function oxygen mask. WO2005/118036 describes masks, with emphasis on scavenging drugs. WO2008/116165 describes systems for delivery of medications using purified air at a positive pressure with delivery coordinated in time with the respiratory cycle of the user.

US2014/0251333 relates to exhalation scavenging.

The invention addresses the problem of achieving effective aerosol treatment with reduction or elimination of gas losses, particularly for high flow treatment.

### Summary of the Disclosure

The invention provides an aerosol treatment system for high flow therapy, as set out in claim 1, and optional features are set out in claims 2-14.

### Detailed Description of the Invention

The invention will be more clearly understood from the following description of some embodiments thereof, given by way of example only with reference to the accompanying drawings in which:
Figs. 1 to 4 are perspective views of an aerosol high flow mask being applied in four steps, respectively;
Fig. 5 is a diagram of a mask in position on a demonstration patient head model used for testing;
Fig. 6 is a plot of bench test results showing particle numbers vs. time determined by an aerodynamic particle analyser for the set-up of Fig. 5 and 50LPM High Flow therapy, and Fig. 7 is a plot of bench test results showing particle measurements with time for various high flow rates, also for the set-up of Fig. 5 and in this case showing in more detail the plots for the higher-level extraction of 70 to 135 LPM;
Fig. 8 is a plot, also for this set-up, showing percentage of particles captured with extraction flow rate;
Fig. 9 shows fluid flows detected by imaging sensors, coded to illustrate volumetric velocity within the mask for nominal mask fit, lower mask fit, and closed mask fit;
Fig. 10 is a set of plots of extraction flowrate with time for a set-up as shown in Fig. 5 and with a nasal pressure sensor added;
Fig. 11 is a histogram of peak nasal pressure for extraction being turned on and off, and Fig. 12 is a histogram of minimum nasal pressure for extraction turned on and off;
Fig. 13 is a diagram showing the mask of Figs. 1 to 4 in place, together with a controller;
Fig. 14 is a flow diagram showing how reduced flow is achieved during inhalation and the aerosol chamber limb is bypassed during inhalation;
Fig. 15 is a pair of plots showing dynamic extraction and pressure monitoring, showing relationship between extraction and breath flows, in which extraction is matched to aerosol flow; and
Fig. 16 is a diagram showing flows of humidified and heated air and O₂ towards the mask and extracted humid breath which is dried in the extraction direction by virtue of a heat recovery condenser and a heat exchanger which recovers heat for humidification.

### Description of the Embodiments

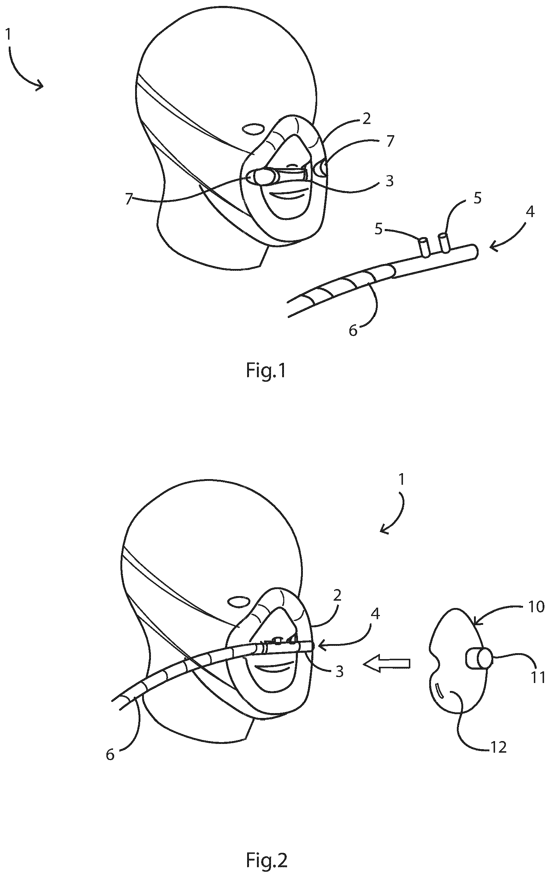

Referring to Fig. 1 a patient interface 1 comprises an annular soft seal base 2 configured to fit around a patient's mouth and nose with a large contact surface of resilient material which is suited to skin contact. The base 2 has a relatively rigid core surrounded on at least the patient side with soft material for patient facial contact. The base spine supports a prong receiver 3 extending across the base 2, and being of a plastics material having a rugged strength to support attachment of a nasal prong head 4 with prongs 4 at the end of tubing 6 for delivery of high flow gas and aerosol to the nostrils.

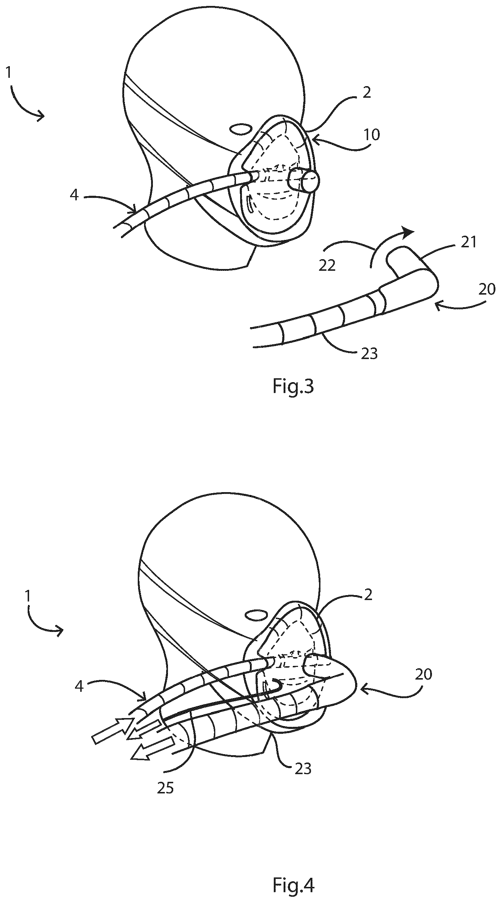

Fig. 2 shows that the interface also includes a shell 10 which fits with a seal to the base 2 and the support 3 to form an enclosed volume around the patient's mouth and nasal area. Fig. 3 shows an extraction head 20 which has an L-shaped conduit 21 with a flange 22 configured to fit to a cylindrical port 11 of the shell.

Fig. 4 shows the full interface 1, with all of these components in place, together with a lead 25 from a pressure sensor within the volume formed by the base 2 and the shell 10. The pressure sensor is attached to the inside surface of the shell 10.

The patient interface is modular, the soft seal base 2 attaching to the patient's head with securing head straps 8. The support 3 supports the prong head 4 with the prongs 5 correctly aligned. It is envisaged that in other embodiments the prong support is self-supporting by way of head straps rather than being attached to the soft seal base, especially for uses without extraction.

The soft seal base 2 is placed on the face first and a comfortable sealing surface is established. This establishes a gas-tight seal and provides a support mechanism for the high flow therapy tubing, and the clinician can conveniently and accurately set up the patient's nasal prongs 3 secured to the soft seal base 2. The fit of the prongs 5 can be checked and adjusted.

The shell 10 can now be assembled. The perimeter of the shell 10 interfaces with the base such that it self-locates and forms a seal, the soft seal base 2 providing a means of securing the shell 10 in place by for example elastic straps, hook-and-loop fasteners, or clips. It is preferred that the shell fit by way of a snap-fit connection with resilient edges. The shell 10, nasal prongs 4/5, and the soft seal base 2 are profiled such that the high flow therapy tubing and head 4 can enter from either the left or the right, and a seal is still established without the need for an additional part. In another embodiment an additional capping feature can be provided.

The shell 10 preferably includes one or more vents to prevent an excessive negative pressure drop within the volume formed by the base 2 and the shell 10 due to extraction. These are located away from exhalation / exhaust airstreams of the mouth and the nostrils. The shell 10 has a port 11 to attach a means of suction, in this case the extraction head 20. The position of the extraction port 11 is such that in use it is opposed to the mouth and nostrils for optimal collection of exhaled / exhausted gasses and particles. The vent or vents may not be in the mask itself, and could for example be part of an exhalation tube. The benefit of a vent is that, because the mask is very effective at sealing the space around the nose and mouth, the operation of the high flow system and the forced extraction system does not cause the system to be too intrusive by acting effectively akin to a ventilator, in which all inhalation and exhalation is controlled. There may for example be a very soft opening on an inhale valve to not affect breathing, and/or a pressure release valve for safely in case of reduced extraction. The vent may have a suitable filter to block outflow of unwanted droplets to avoid contamination of the environment.

The shell also has a retainer to attach a sensor for measuring the internal mask pressure.

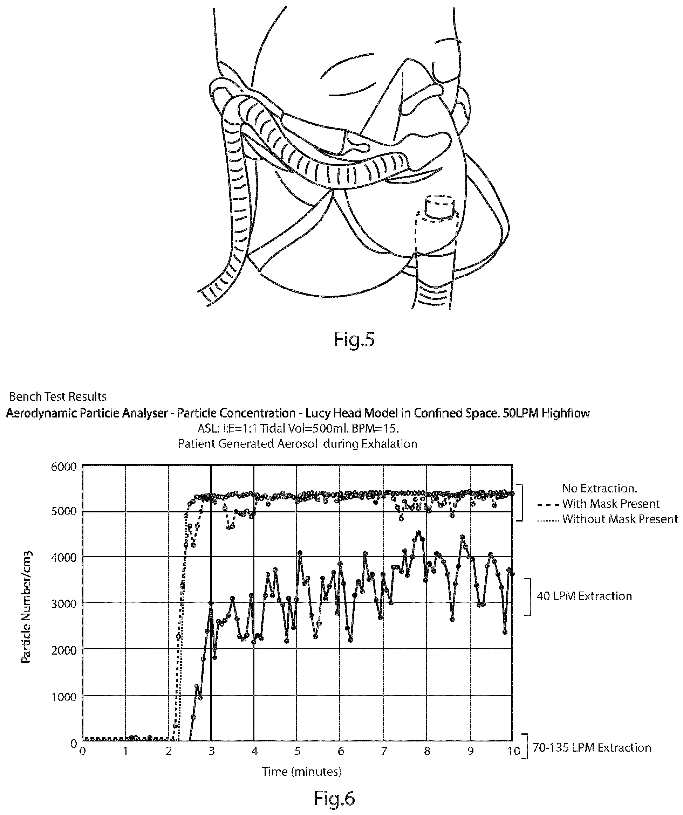

Figs. 6 to 8 shows test results for ambient aerosol particles in the environment surrounding the patient's head for with the mask and system shown in Fig. 5. This mask is conventional in terms of how it is configured to engage the face, simply a flexible clear polymer with holes cut to take the HFNT delivery and extraction hoses. It does not have the benefits of the mask of Figs. 1 to 4.

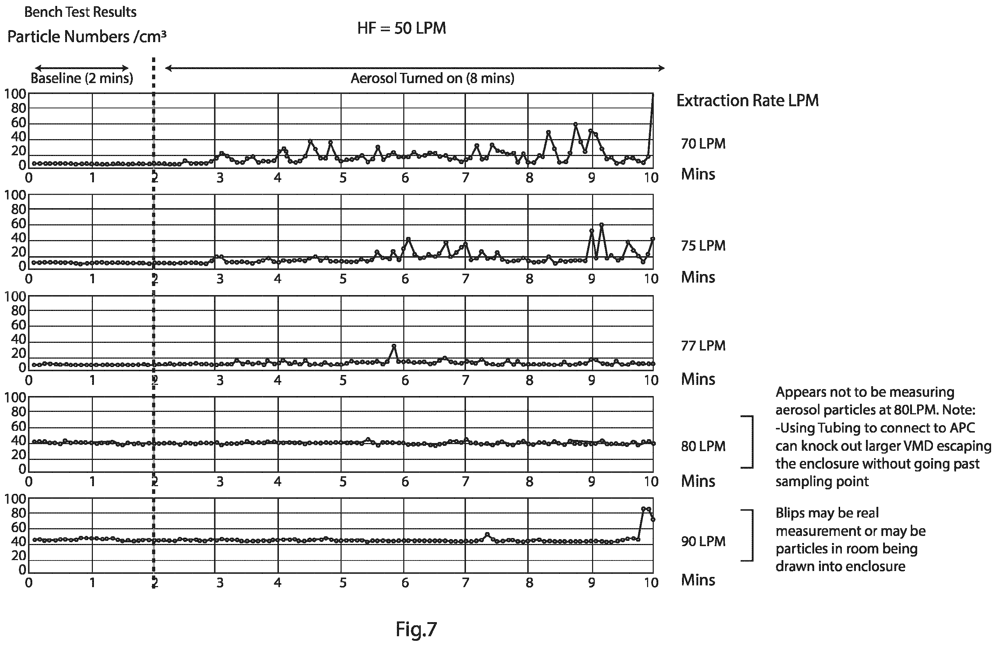

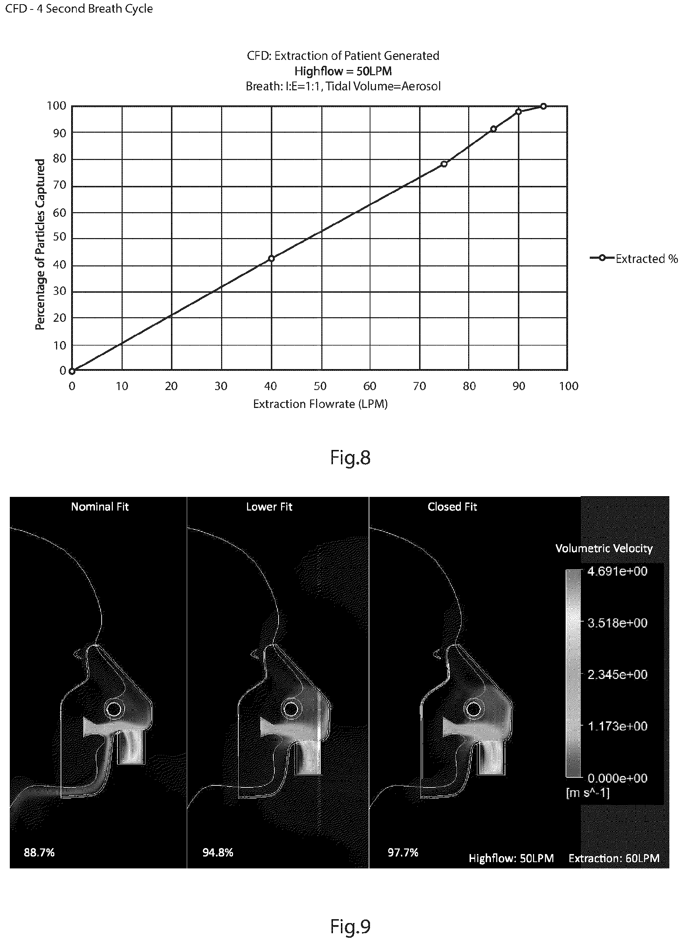

Fig. 6 demonstrates an extraction flowrate in the region of 80LPM to 100LPM is required to capture approximately all aerosol particles, in these examples with treatment at 50LPM. Fig. 6 demonstrates the major differences between the bands of 7- to 135 LPM, 40 LPM, and no extraction. In the latter there is essentially no difference between situations where the mask is present and is not, because of losses around the edges of the mask. Fig. 7 shows more detail for the higher extraction rate band, with the vertical axes showing particles in the tens per cm³. Fig. 9 is a summary histogram showing that there is a liner relationship between extraction flowrate and percentage of particles captured., reaching full capture at about 90LPM extraction.

The positioning of the extraction port opposite the patient's mouth affects the rate of emission capture for a given extraction rate. In Fig. 9, the 'Lower Fit' outperforms the 'Nominal Fit' in terms of capture of emissions (94.8% vs. 88.7%). The degree of sealing between the mask and the face affects rate of emission capture for a given extraction rate. In Fig. 9 'Closed Fit' is the same as 'Lower Fit' except that there is sealing at the cheeks. At the same level of extraction (60 LPM) versus the same emission flowrate (50LPM, there is no breath in this example) the 'Closed Fit' outperforms the 'Lower Fit' in term of capture of emissions (97.7% vs. 94.8%).

The effect of extraction on the nasal cavity has been investigated. A test setup as shown in Fig. 5 but with addition of a nasal pressure sensor involved:
Delivery of humified high flow @ 50LPM
Pressure tube inserted into nasal cavity
Extraction flowrate either Off or @100LPM
Flowmeter in-line with extraction source.

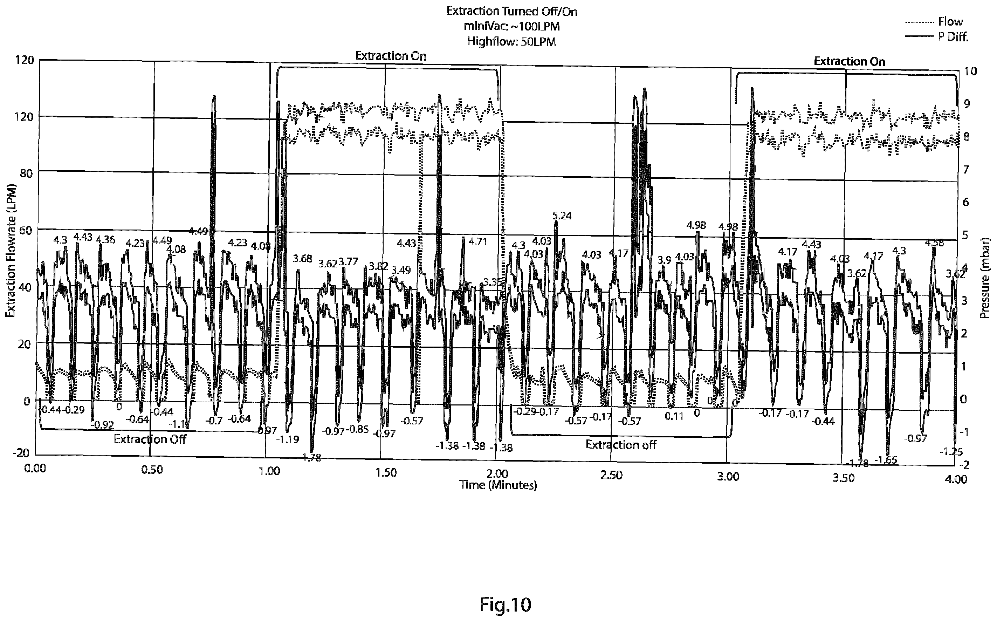

The results of this are illustrated in Figs. 10 to 12, which displays the distribution of the nasal pressure peaks, which occur during exhalation, with and without extraction. The average difference is a reduction of 0.3mBar when extraction is applied; the difference of the maximums is 0.53mBar.

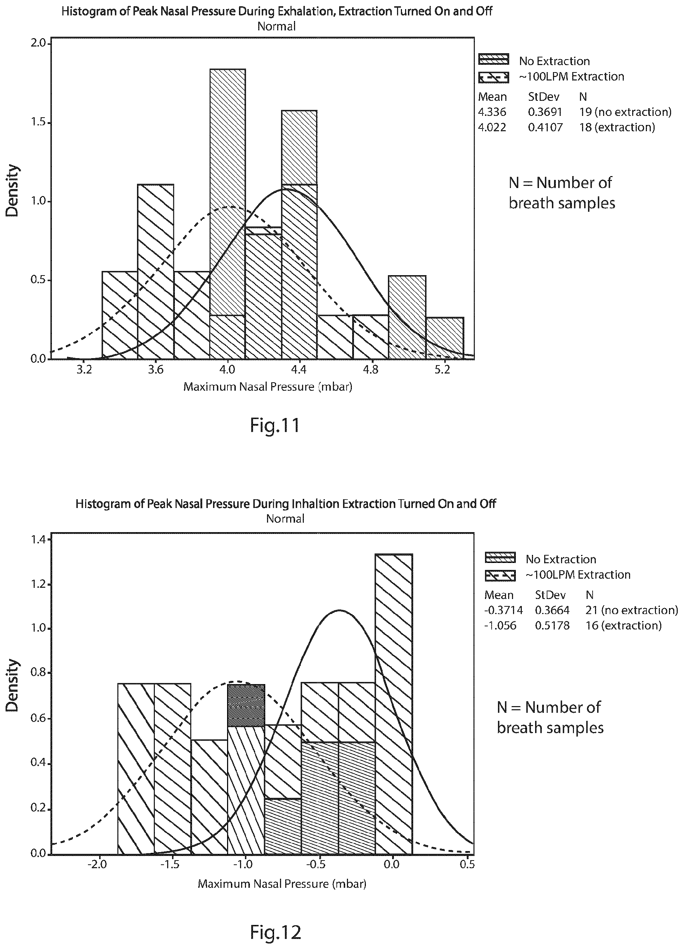

Fig. 12 displays the distribution of the nasal pressure troughs (these occur during inhalation) with and without extraction. The average difference is a reduction of 0.68mBar when extraction is applied; the difference of the minimums is also 0.68mBar.

These results demonstrate the advantages of decreasing extraction during exhalation and not having any extraction during inhalation.

Major advantages of the invention include:
Flexible patient-engaging side of the base 2 provides consistent sealing, especially allowing successful extraction at lower flow rates.
A predictable seal allows less safety factor buffer on the applied extraction rate.
Less extraction allows less pressure drop, less noise, more extraction source options, controlled vent ports.
Maximise extraction for a given extraction flowrate.
Avoidance of occlusion with patient's face.
Reduced wind noise / wind feeling
Sized to alleviate pressure drop by a predictable quantity
Modularity, allowing fitting of the base to be secured to head and adjusted for sealing and comfort independent of the therapy.
Allows clinical access to setup / check/ change prongs.
Pressure Port.
Provides means of measuring pressure internal to the mask.
Can be used as a safety measure.
Can be used to detect the breath and dynamically control the extraction rate.
Allows for more effective extraction with reduced impact on pressure drop.

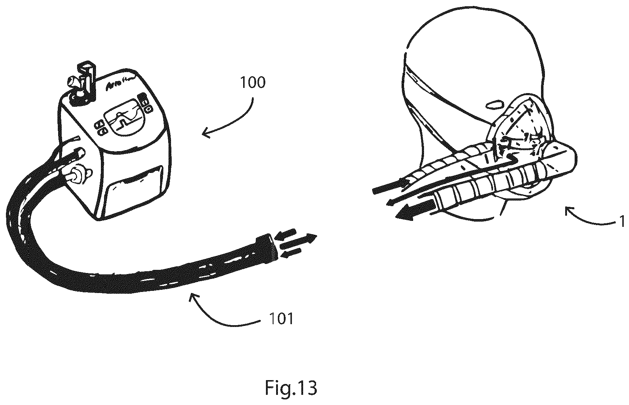

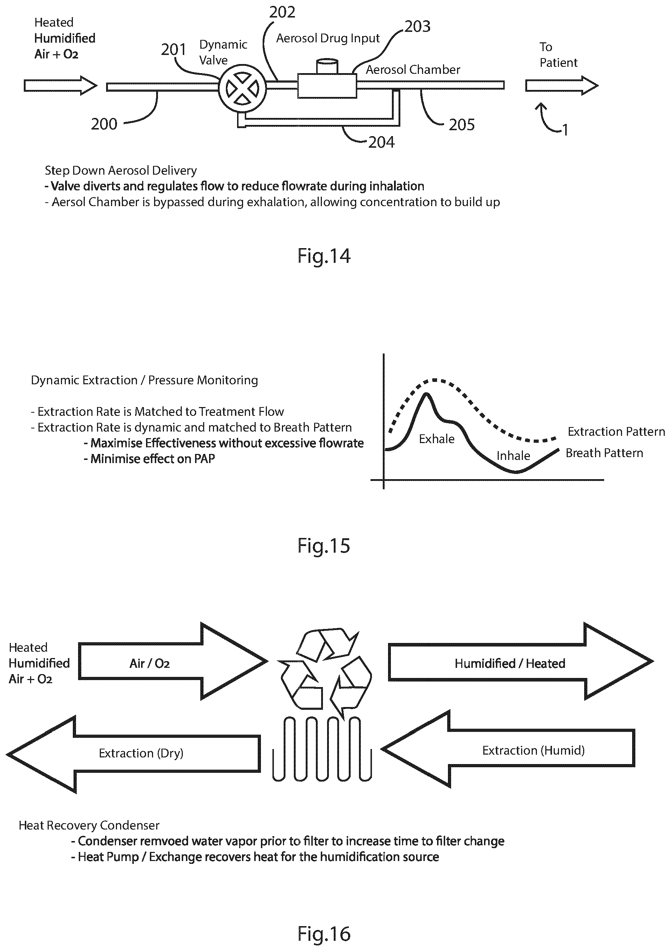

The patient interface 1 can connect to a standalone aerosol / high flow therapy device 100 by a tubing set 101 as depicted in Fig. 13. This allows breath-synchronised step-down aerosol delivery. As illustrated in Fig. 14 a heated humidified air/ O₂ mixture is delivered on a tube 200, and flow is split by a valve 201 into an aerosol branch 202 with a nebulizer 203 and a parallel bypass branch 204. The branches merge into a common tube 205 which leads to the interface 1.

In some examples, the aerosol delivery path can include an aerosol chamber having an increased volume to slow down the flowrate at the point of aerosol delivery

### Bypass Scenarios

The valve 201 can dynamically throttle the flowrate, and can dynamically divert the flowrate to provide scenarios such as:
Full bypass, normal high flow treatment with no aerosol.
No bypass, open throttle, continuous aerosol delivery.
No bypass, open throttle, breath synchronised aerosol delivery (pressure sensor used to detect breath pattern).
Breath-synchronised bypass: divert through bypass during exhalation. Nebuliser is continuously on allowing aerosol to accumulate in the chamber. During inhalation divert the flow through the chamber for increased aerosol concentration.

### Step-Down Delivery

Step-down aerosol delivery: when switching to the aerosol path 202 during inhalation, the average flowrate is reduced. This will improve dose efficiently. The period of reduced flowrate is short to prevent de-recruitment effects. There is a ramp down / ramp up of the reduced flowrate, and these ramps can be controlled / modulated to minimise de-recruitment and discomfort.

### Dynamic Extraction / Pressure Monitoring

The system controller can adapt the baseline extraction to match the high flow therapy setting. The controller can be programmed to dynamically change the extraction rate to match the breath pattern, as illustrated in Fig. 15. This can maximise the effectiveness of extraction, and therefore reduce the required extraction rate. This has the benefit of reducing the impact on the treatment pressure, and also reduces the extraction source requirements.

### Filtration

An advantageous part of the extraction system is that there is filtration in line with the extracted airflow to capture any pathogens or drug before it is vented to the ambient room. This can be a standard commercial filter that can be changed out by the clinicians. Due to the large levels of humidity in the expelled gas, the filter will become saturated, and the filter regime adapted accordingly. The system can determine the actual flowrate based off the mask pressure readings. Or, additional flow and pressure sensors could be employed on the system side of the filter. The system can increase the power supplied to the extraction source to keep the extraction flowrate consistent over time as the filter approaches saturation.

### Condenser

A condenser can be employed to take vapour out of the extracted gas prior to it reaching the filter. This can prolong the life of the filter. The condensing mechanism is preferably such that the surfaces that make contact with the extracted gasses are part of a disposable circuit. A heat pump (for example using a Peltier heat exchanger) can be employed to increase the rate of condensing, as illustrated in Fig. 16. The heat collected in this exchange can be employed in heating of the high flow therapy delivered to the patient. This would have energy saving benefits.
No Fugitive emissions of treatment drug
No spreading of patient pathogens
Increased drug efficiency
Increased drug rate

The invention is not limited to the embodiments described but may be varied in construction and detail, within the scope of the claims. For example, it is envisaged that the mask is provided as a pre-assembled component, perhaps using a sizing chart to allow a clinician to preconfigure and position nasal prongs. The mask, if it does not have a removable shell may have an access flap to allow adjustment of the nasal prongs. The performance features and advantages described for an interface having conventional features apply to an interface of Figs. 1 to 4, and it is expected that performance would be better because of the enhanced sealing and other advantages described with reference to Figs. 1 to 4. Irrespective of the interface used, any interface which supports the HFNT delivery and extraction and provides a closed or near-closed environment around the mouth and nose is advantageous as described above with reference to Figs. 5 to 12 and 14 to 16, and where the interface of Figs. 1 to 4 and 13 is used it enhances the closed nature of this volume around the nose and mouth.

## Claims

1. An aerosol treatment system comprising:
a patient interface (1) to cover a patient's mouth and nose a high flow treatment system linked with the patient interface (1),
an aerosol delivery apparatus (4, 6), an extraction apparatus (20),
a tube (200) for delivery of a heated humidified air/ O₂ mixture, an aerosol branch (202) with a nebulizer (203), a parallel bypass branch (204), a common tube (205) which leads to the interface (1), wherein the aerosol branch (202) and the bypass branch (204) merge into the common tube (205),
a valve (201) arranged to split delivery flow into the aerosol branch (202) with the nebulizer (203) and the parallel bypass branch (204),
sensors for detecting patient breathing, and
a controller configured to:
control delivery of aerosol and gas to the patient interface (1), extract gases from a volume enclosed by the patient interface, and to provide breath-synchronised delivery.

2. An aerosol treatment system of claim 1, wherein the high flow treatment system is a high flow nasal treatment system (HFNT).

3. An aerosol treatment system as claimed in either of claims 1 or 2, wherein the system comprises a heater and a humidifier, separately or combined, to provide the heated humidified air/ O2 mixture (200) delivered to the interface (1).

4. An aerosol treatment system as claimed in any of claims 1 to 3, wherein the controller is configured to dynamically control the system to provide scenarios including one or more of:
a full bypass;
no bypass with continuous aerosol delivery;
no bypass with breath synchronised aerosol delivery according to signals from a pressure sensor in the volume enclosed by the interface; and/or
breath-synchronised bypass with divert through the bypass during exhalation; continuously-on aerosol generation allowing aerosol to accumulate in a chamber and during inhalation divert the flow through the chamber for increased aerosol concentration.

5. An aerosol treatment system as claimed in any of claims 1 to 4, wherein the aerosol generator includes a chamber having an increased volume to slow down the delivery flowrate; and wherein the controller is configured to provide step-down aerosol delivery; and wherein the controller is configured to reduced gas flowrate and increased aerosol delivery during inhalation to improve dose efficiently.

6. An aerosol treatment system as claimed in any preceding claim, wherein the interface comprises:
a base (2) configured to surround at least part of a patient's mouth and nose and engage the skin with a resilient seal,
a support (3) on the base for supporting an aerosol or gas delivery head,
a shell (10) configured to form an enclosure together with the base, and
an extraction port (11) for attachment of an extraction system to extract gas from said volume in use.

7. An aerosol treatment system as claimed in claim 6, wherein the base (2) is annular, configured to fully surround the patient's mouth and nose; wherein the base (2) comprises a spine on which there is an inner soft layer for engaging the patient's face; wherein the support (3) is mounted to the spine; and wherein the support (3) extends across the base at or adjacent a central location to bisect the base.

8. An aerosol treatment system as claimed in claim 7, wherein the support (3) comprises openings to receive nasal prongs (5) of an aerosol delivery head (4); and wherein the shell has at least one opening for passage of an aerosol delivery tube.

9. An aerosol treatment system as claimed in claim 8, wherein the shell comprises a pair of openings to allow connection of an aerosol head at either side.

10. An aerosol treatment system as claimed in claim 9, wherein the shell comprises blanks to seal off an un-used opening.

11. An aerosol treatment system as claimed in any of claims 6 to 10, wherein the shell is configured to snap-fit to the base.

12. An aerosol treatment system as claimed in any of claims 6 to 11, wherein the extraction port is located at a location approximately central to the base for alignment in use with a patient's mouth.

13. An aerosol treatment system as claimed in any of claims 6 to 12, wherein said sensors comprise a pressure sensor (25) mounted to the shell.

14. An interface as claimed in any of claims 6 to 13, wherein the shell includes at least one vent.

## Patentansprüche

1. Aerosolbehandlungsystem, umfassend:
eine Patientenschnittstelle (1), um Mund und Nase eines Patienten abzudecken,
ein High-Flow-Behandlungssystem, das mit der Patientenschnittstelle (1) gekoppelt ist,
eine Aerosolzuführvorrichtung (4, 6)
eine Extraktionsvorrichtung (20),
einen Schlauch (200) zur Zufuhr eines erwärmten befeuchteten Luft-O₂-Gemischs, einen Aerosolzweig (202) mit einem Vernebler (203), einen Zweig (204) zur parallelen Umgehung, einen Sammelschlauch (205), der zu der Schnittstelle (1) führt, wobei der Aerosolzweig (202) und der Umgehungsschlauch (204) in den Sammelschlauch (205) zusammenlaufen,
ein Ventil (201), das dazu angeordnet ist, einen Zufuhrstrom in den Aerosolzweig (202) mit dem Vernebler (203) und den Zweig (204) zur parallelen Umgehung zu teilen, Sensoren zum Detektieren der Atmung des Patienten und
eine Steuereinheit, die dazu konfiguriert ist:
die Zufuhr von Aerosol und Gas zu der Patientenschnittstelle (1) zu steuern,
Gase aus einem von der Patientenschnittstelle umschlossenen Volumen zu extrahieren und atemsynchronisierte Zufuhr bereitzustellen.

2. Aerosolbehandlungssystem nach Anspruch 1, wobei das High-Flow-Behandlungssystem ein System zur nasalen High-Flow-Behandlung (High Flow Nasal Treatment System, HFNT) ist.

3. Aerosolbehandlungssystem nach entweder Anspruch 1 oder 2, wobei das System eine Heizvorrichtung und einen Befeuchter umfasst, die getrennt oder kombiniert sind, um das der Schnittstelle (1) zugeführte erwärmte befeuchtete Luft-O₂-Gemisch (200) bereitzustellen.

4. Aerosolbehandlungssystem nach einem der Ansprüche 1 bis 3, wobei die Steuereinheit dazu konfiguriert ist, das System dynamisch zu steuern, um Szenarios bereitzustellen, die eines oder mehrere der Folgenden umfassen:
eine vollständige Umgehung;
keine Umgehung bei kontinuierlicher Aerosolzufuhr;
keine Umgehung bei atemsynschronisierter Aerosolzufuhr gemäß Signalen von einem Drucksensor in dem von der Schnittstelle eingeschlossenen Volumen; und/oder
atemsynchronisierte Umgehung mit Umleitung durch die Umgehung hindurch während Exhalation;
kontinuierlich aktive Aerosolerzeugung, die ermöglicht, dass sich Aerosol in einer Kammer ansammelt, und während Inhalation Umleitung des Flusses durch die Kammer hindurch zur erhöhten Aerosolkonzentration.

5. Aerosolbehandlungssystem nach einem der Ansprüche 1 bis 4, wobei der Aerosolerzeuger eine Kammer mit einem erhöhten Volumen zum Verlangsamen des Zufuhrvolumenstroms aufweist; und wobei die Steuereinheit dazu konfiguriert ist, Step-Down-Aerosolzufuhr bereitzustellen; und wobei die Steuereinheit zu verringertem Gasvolumenstrom und erhöhter Aerosolzufuhr während der Inhalation konfiguriert ist, um die Dosiseffizienz zu verbessern.

6. Aerosolbehandlungssystem nach einem der vorangehenden Ansprüche, wobei die Schnittstelle Folgendes umfasst:
eine Basis (2), die dazu konfiguriert ist, mindestens eine Teil des Munds und der Nase eines Patienten zu umgeben und mit einer elastischen Dichtung mit der Haut in Eingriff zu kommen,
eine Halterung (3) an der Basis zum Halten eines Aerosol- oder Gaszuführkopfs,
eine Schale (10), die dazu konfiguriert ist, zusammen mit der Basis ein Gehäuse zu bilden, und
einen Extraktionsanschluss (11) zum Anbringen eines Extraktionssystems, um im Gebrauch Gas aus dem Volumen zu extrahieren.

7. Aerosolbehandlungssystem nach Anspruch 6, wobei die Basis (2) ringförmig ist, und dazu konfiguriert ist, Mund und Nase des Patienten vollständig zu umgeben; wobei die Basis (2) ein Rückgrat umfasst, auf dem sich eine innere weiche Schicht zum Eingriff mit dem Gesicht des Patienten befindet; wobei die Halterung (3) an dem Rückgrat befestigt ist, und wobei sich die Halterung (3) an einer mittleren Stelle oder dieser benachbart über die Basis erstreckt, um die Basis in zwei zu teilen.

8. Aerosolbehandlungssystem nach Anspruch 7, wobei die Halterung (3) Öffnungen zum Aufnehmen von Nasenstutzen (5) eine Aerosolzuführkopfs (4) umfasst; und wobei die Schale mindestens eine Öffnung zum Hindurchgelangen eines Aerosolzuführschlauchs aufweist.

9. Aerosolbehandlungssystem nach Anspruch 8, wobei die Schale ein Paar Öffnungen umfasst, um das Anschließen eines Aerosolkopfs an beiden Seiten zu ermöglichen.

10. Aerosolbehandlungssystem nach Anspruch 9, wobei die Schale Stopfen zum Verschließen einer ungenutzten Öffnung umfasst.

11. Aerosolbehandlungssystem nach einem der Ansprüche 6 bis 10, wobei die Schale zur Schnappverbindung mit der Basis konfiguriert ist.

12. Aerosolbehandlungssystem nach einem der Ansprüche 6 bis 11, wobei sich der Extraktionsanschluss an einer Stelle ungefähr mittig in Bezug auf die Basis befindet, um im Gebrauch auf den Mund eines Patienten ausgerichtet zu sein.

13. Aerosolbehandlungssystem nach einem der Ansprüche 6 bis 12, wobei die Sensoren einen an der Schale befestigten Drucksensor (25) umfassen.

14. Schnittstelle nach einem der Ansprüche 6 bis 13, wobei die Schale mindestens eine Entlüftungsöffnung umfasst.

## Revendications

1. Système d'aérosolisation comprenant :
une interface patient (1) permettant de couvrir le nez et la bouche d'un patient,
un système de traitement à haut débit lié à l'interface patient (1),
un appareil de distribution aérosol (4, 6),
un appareil d'extraction (20),
un tube (200) pour la distribution d'un mélange air/O₂ humidifié chauffé, une branche d'aérosol (202) avec un nébuliseur (203), une branche de dérivation parallèle (204), un tube commun (205) qui mène à l'interface (1), dans lequel la branche d'aérosol (202) et la branche de dérivation (204) fusionnent dans le tube commun (205),
une soupape (201) conçue pour diviser l'écoulement dans la branche d'aérosol (202) avec le nébuliseur (203) et la branche de dérivation parallèle (204),
des capteurs permettant de détecter une respiration du patient, et
un dispositif de commande configuré pour :
commander la distribution d'aérosol et de gaz à l'interface patient (1),
extraire des gaz d'un volume contenu par l'interface patient, et pour fournir une distribution à synchronisation respiratoire.

2. Système d'aérosolisation selon la revendication 1, dans lequel le système de traitement à haut débit est un système de traitement nasal à haut débit (HFNT).

3. Système d'aérosolisation selon l'une quelconque des revendications 1 ou 2, dans lequel le système comprend un appareil de chauffage et un humidificateur, séparés ou combinés, pour fournir le mélange air/O₂ humidifié chauffé (200) distribué à l'interface (1).

4. Système d'aérosolisation selon l'une quelconque des revendications 1 à 3, dans lequel le dispositif de commande est configuré pour commander dynamiquement le système pour fournir des scénarios incluant un ou plusieurs des éléments suivants :
une dérivation totale ;
aucune dérivation avec une distribution d'aérosol continue ;
aucune dérivation avec une distribution d'aérosol à synchronisation respiratoire selon des signaux provenant d'un capteur de pression dans le volume contenu par l'interface ; et/ou
une dérivation à synchronisation respiratoire avec détournement à travers la dérivation pendant l'exhalation ; une génération d'aérosol en continu permettant à l'aérosol de s'accumuler dans une chambre et pendant l'inhalation de détourner le débit à travers la chambre pour une concentration d'aérosol augmentée.

5. Système d'aérosolisation selon l'une quelconque des revendications 1 à 4, dans lequel le générateur d'aérosol inclut une chambre présentant un volume augmenté pour ralentir le débit de distribution ; et dans lequel le dispositif de commande est configuré pour fournir une distribution d'aérosol en descente ; et dans lequel le disposition de commande est configuré pour un débit de gaz réduit et une distribution d'aérosol augmentée pendant l'inhalation pour améliorer l'efficacité de la dose.

6. Système d'aérosolisation selon l'une quelconque des revendications précédentes, dans lequel l'interface comprend :
une base (2) configurée pour entourer au moins une partie de la bouche et du nez d'un patient et mettre la peau en contact avec un joint d'étanchéité élastique,
un support (3) sur la base permettant de supporter une tête de distribution d'aérosol ou de gaz,
une coque (10) configurée pour former une enceinte conjointement avec la base, et
un orifice d'extraction (11) permettant la fixation d'un système d'extraction pour extraire un gaz dudit volume lors de l'utilisation.

7. Système d'aérosolisation selon la revendication 6, dans lequel la base (2) est annulaire, configurée pour entourer complètement la bouche et le nez du patient ; dans lequel la base (2) comprend une colonne vertébrale sur laquelle se trouve une couche souple interne pour l'entrée en contact avec le visage du patient ; dans lequel le support (3) est monté sur la colonne vertébrale ; et dans lequel le support (3) s'étend à travers la base au niveau de ou adjacent à un emplacement central pour couper la base en deux parties égales.

8. Système d'aérosolisation selon la revendication 7, dans lequel le support (3) comprend des ouvertures pour recevoir des pinces nasales (5) d'une tête de distribution d'aérosol (4) ; et dans lequel la coque présente au moins une ouverture pour le passage d'un tube de distribution d'aérosol.

9. Système d'aérosolisation selon la revendication 8, dans lequel la coque comprend une paire d'ouvertures pour permettre une connexion d'une tête d'aérosol au niveau de chaque côté.

10. Système d'aérosolisation selon la revendication 9, dans lequel la coque comprend des flans pour condamner une ouverture inutilisée.

11. Système d'aérosolisation selon l'une quelconque des revendications 6 à 10, dans lequel la coque est configurée pour s'encliqueter sur la base.

12. Système d'aérosolisation selon l'une quelconque des revendications 6 à 11, dans lequel l'orifice d'extraction est situé au niveau d'un emplacement plus ou moins central à la base pour l'alignement lors de l'utilisation avec la bouche d'un patient.

13. Système d'aérosolisation selon l'une quelconque des revendications 6 à 12, dans lequel lesdits capteurs comprennent un capteur de pression (25) monté sur la coque.

14. Interface selon l'une quelconque des revendications 6 à 13, dans laquelle la coque inclut au moins un évent.
